# EUROPEAN PATENT SPECIFICATION

(11) **EP 2 114 884 B1**
(45) Date of publication and mention of the grant of the patent: **15.12.2010**
(21) Application number: 07750606.1
(22) Date of filing: 09.02.2007
(51) Int. Cl.: C07D 213/32, C07D 213/80

(54) **PROCESS FOR THE PREPARATION OF 2-SUBSTITUTED-5-(1-ALKYLTHIO) ALKYLPYRIDINES**
VERFAHREN ZUR HERSTELLUNG VON 2-SUBSTITUIERTEN 5-(1-ALKYLTHIO)ALKYLPYRIDINEN
PROCÉDÉ DE PRÉPARATION DE 2-5-(1-ALKYLTHIO)ALKYLPYRIDINES SUBSTITUÉES

(43) Date of publication of application: 11.11.2009
(73) Proprietor: Dow AgroSciences LLC, Indianapolis IN 46268-1054 (US)
(72) Inventor: MEYER, Kevin, G., Zionsville, IN 46077 (US); ARNDT, Kim, E., Carmel, IN 46032 (US)
(74) Representative: Dey, Michael
(86) International application number: PCT/US2007/003779
(87) International publication number: WO 2008/097234

(56) References cited:
- GB-A- 2 192 877
- US-A1- 2005 228 027

## Description

The present invention concerns processes for preparation of 2-substituted-5-(1-alkylthio)alkylpyridines.

The 2-substituted-5-(1-alkylthio)alkylpyridines are useful intermediates for the preparation of certain new insecticides; see, for example, U.S. Patent Publication 2005/0228027. It would be advantageous to produce 2-substituted-5-(1-alkylthio)alkylpyridines efficiently and in high yield. Known from GB-A-2 192 877 are pyridine derivatives which differ from the present compounds in the substituent R⁴ (cf. below).

One aspect of the present invention concerns a process for the preparation of 2-substituted-5-(1-alkylthio)alkylpyridine (I), 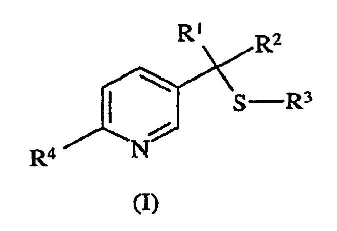 wherein
R¹ and R² independently represent H, C₁-C₄ alkyl, or either of R¹ or R² taken together with R³ represent a 4- to 6-membered saturated ring, or R¹ taken together with R² represent a 3- to 6-membered saturated ring optionally substituted with an O or a N atom;
R³ represents C₁-C₄ alkyl or R³ taken together with either of R¹ or R² represent a 4- to 6-membered saturated ring; and
R⁴ represents C₁-C₄ alkyl or C₁-C₄ haloalkyl;
which comprises:
a) condensing a substituted enone (II), 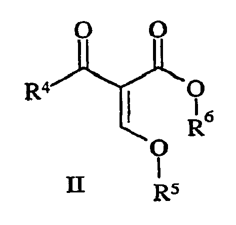 wherein
   R⁴ is as previously defined; and
   R⁵ and R⁶ independently represent C₁-C₄ alkyl;
   with an enamine (flD, 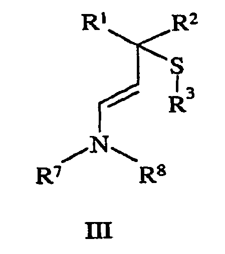 wherein
   R¹, R⁷ and R³ are as previously defined; and
   R⁷ and R⁸ independently represent C₁-C₄ alkyl or R⁷ and R⁸ taken together with N represent a 5-membered saturated or unsaturated ring;
b) cyclizing the reaction mixture from step a) in the presence of ammonia or a reagent capable of generating ammonia to produce a 2,3,5-substituted pyridine (IV), 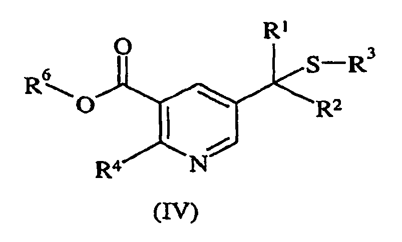 wherein
   R¹, R², R³, R⁴ and R⁶ are as previously defined; and
c) saponifying and decarboxylating the 2,3,5-substituted pyridine (IV) to give 2-substituted-5-(1-alkylthio)alkylpyridine (I). This method is particularly well suited to prepare compounds in which R⁴ represents CF₃.

Another aspect of the invention concerns the intermediate nicotinic acid derivatives of the Formula (IV) 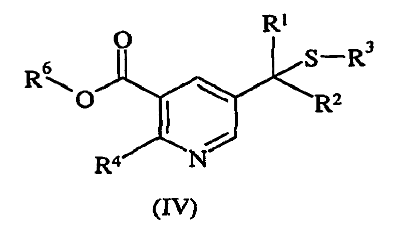 wherein
R¹ and R² independently represent H, C₁-C₄ alkyl, or either of R¹ or R² taken together with R³ represent a 4- to 6-membered saturated ring, or R¹ taken together with R² represent a 3- to 6-membered saturated ring optionally substituted with an O or a N atom;
R³ represents C₁-C₄ alkyl or R³ taken together with either of R¹ or R² represent a 4- to 6-membered saturated ring;
R⁴ represents C₁-C₄ alkyl or C₁-C₄ haloalkyl;; and
R⁶ represents H or C₁-C₄ alkyl.

Unless specifically limited otherwise, the term "alkyl" (including derivative terms such as "haloalkyl"), as used herein, include straight chain, branched chain, and cyclic groups. Thus, typical alkyl groups are methyl, ethyl, 1-methylethyl, propyl, 1,1-dimethylethyl, and cyclopropyl. The term "halogen" includes fluorine, chlorine, bromine and iodine. The term "haloalkyl" includes alkyl groups substituted with any number from one to the maximum possible number of halogen atoms.

One aspect of the present invention concerns a process for the preparation of 2-substituted-5-(1-alkylthio)alkylpyridine (I), 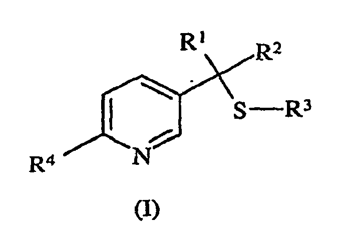 wherein
R¹ and R² independently represent H, C₁-C₄ alkyl, or either of R¹ or R² taken together with R³ represent a 4- to 6-membered saturated ring, or R¹ taken together with R² represent a 3- to 6-membered saturated ring optionally substituted with an O or a N atom;
R³ represents C₁-C₄ alkyl or R³ taken together with either of R¹ or R² represent a 4- to 6-membered saturated ring; and
R⁴ represents C₁-C₄ alkyl or C₁-C₄ haloalkyl;
which comprises:
a) condensing a substituted enone (II), 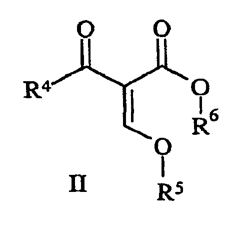 wherein
   R⁴ is as previously defined; and
   R⁵ and R⁶ independently represent C₁-C₄ alkyl;
   with an enamine (III), 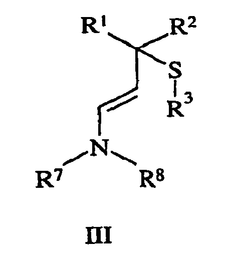 wherein
   R¹, R² and R³ are as previously defined; and
   R⁷ and R⁸ independently represent C₁-C₄ alkyl or R⁷ and R⁸ taken together with N represent a 5-membered saturated or unsaturated ring;
b) cyclizing the reaction mixture from step a) in the presence of ammonia or a reagent capable of generating ammonia to produce a 2,3,5-substituted pyridine (IV), 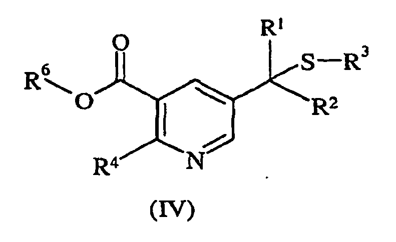 wherein
   R¹, R², R³, R⁴ and R⁶ are as previously defined; and
c) saponifying and decarboxylating the 2,3,5-substituted pyridine (IV) to give 2-substituted-5-(1-alkylthio)alkylpyridine (I).

The substituted enone (II) starting materials are commercially available or can be prepared from the corresponding keto ester substrates and alkylorthoformates. Typically, acetoacetates are condensed with trialkylorthoformates to yield compounds of type (II). Enamines (III) starting materials can be conveniently prepared from the addition of a suitably substituted amine to an appropriately substituted aldehyde in the presence of a water adsorbing material, with or without a suitable solvent. Typically, the appropriate substituted propionaldehyde is reacted with an anhydrous disubstituted amine at -20 °C to 20 °C in the presence of a desiccant such as anhydrous potassium carbonate, and the product is isolated by distillation.

In steps a) and b), approximately equimolar quantities of the substituted enone (II) and the enamine (III) and ammonia are required in the process, although 2-4 fold excesses of the ammonia or the ammonia precursor are often preferred.

Typical reagents capable of generating ammonia include, for example, 1) an ammonium salt of an acid, preferably an organic acid, 2) formamide, or 3) formamide with an acid or acid salt. The ammonium salt of any aliphatic or aromatic organic acid can be used, but for convenience of processing, the ammonium salts of C₁-C₄ alkanoic acids are preferred. Ammonium formate and ammonium acetate are preferred.

Step a) is illustratively conducted in a polar high-boiling solvent that is miscible with water. Preferred solvents include: amides such as formamide, dimethyl formamide, dimethyl acetamide; alcohols such as methanol, ethanol, isopropanol, (2-methoxy)ethanol; and alkylnitriles including acetonitrile.

The reaction is conducted at a temperature from -20°C to 150°C. Temperatures from 0°C to 80°C are preferred.

The product is isolated by conventional techniques such as silica gel chromatography or fractional distillation.

In a typical reaction, the substituted enone (II) and enamine (III) are dissolved in the polar solvent at -5°C to 20°C and agitated until the substituted enone (II) and enamine (III) are consumed. In step b), the ammonium salt of the organic acid is then added, and the mixture is heated at 50°C to 150°C until the reaction is complete. After dissolving in a non-water miscible solvent and washing with water and, optionally, brine, the 2,3,5-substituted pyridine (IV) is isolated by silica gel column chromatography or by vacuum distillation.

In step c), 2,3,5-substituted pyridine (IV) is saponified by well-known procedures with base, preferably an alkali metal hydroxide such as lithium hydroxide, at 0°C to 50°C in a polar solvent that is miscible with water, such as tetrahydrofuran. The resulting pyridine carboxylate salt is neutralized and is then decarboxylaed by well-known procedures such as, for example, by heating in a high boiling solvent such as DowTherm A (available by The Dow Chemical Company), optionally with copper powder, at temperatures between 150°C and 250°C to obtain 2-substituted-5-(1-alkylthio)alkylpyridine (I), which can be isolated by conventional methods such as silica gel chromatography or vacuum distillation.

The following examples are presented to illustrate the invention.

### EXAMPLES

Example 1. Preparation of 5-(1-Methylthioethyl)-2-trifluoromethylpyridine

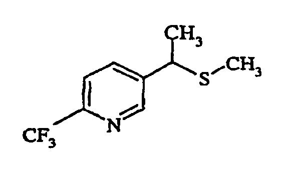

### Step 1. Preparation of 1-(3-Methylthiobut-1-enyl)pyrrolidine

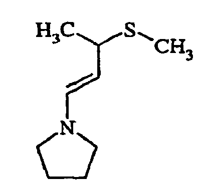

To a dry 5000 milliliter (mL) round bottom flask equipped with mechanical stirrer, nitrogen inlet, addition funnel, and thermometer, was charged 591 g (4.27 moles) of dry granular potassium carbonate and 1428 ml (17.1 moles) of anhydrous pyrrolidine. The mixture was stirred under a atmosphere of nitrogen, and cooled to 4°C with an ice bath, after which 1050 ml (8.9 moles) of 3-methyl-thiobutyraldehyde was added at a rate that maintains the temperature below 10°C. Upon the completion of the addition, the cooling bath was removed and the reaction was allowed to reach room temperature. The reaction contents were then filtered through a sintered glass filter funnel to remove the solids and the solids were washed with 200 ml of anhydrous ethyl ether. The filtrate was concentrated under vacuum on a rotary evaporator until all of the pyrrolidine was removed to afford 1,519 g of 1-(3-methylthiobut-1-enyl)pyrrolidine as a red liquid. ¹H NMR CDCl₃ δ 1.36 (d, 3H), 1.85 (m, 4H), 2.02 (s, 3H), 3.02 (m, 4H), 3.26 (q, 1H), 3.98 (dd, 1H), 6.25 (d, 1H).

Step 2. Preparation of 5-(1-Methylthioethyl)-2-trifluoromethyl-nicotinic acid ethyl ester

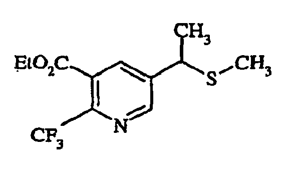

To a dry 50 mL round bottom flask equipped with magnetic stirrer, nitrogen inlet, addition funnel, and thermometer, was charged the 1-(3-methyl-thiobut-1-enyl)pyrrolidine (5.0 g, 0.0291 mol) and 100 mL of dry acetonitrile. The 2-[1-ethoxymethylidene]-4,4,4-trifluoro-3-oxo-butyric acid ethyl ester (7.0 g, 0.0291 mol) was added dropwise, and the reaction was stirred at room temperature for 1 hour. An aliquot was analyzed by gas chromatography (GC) which indicated that no starting material remained. Ammonium acetate (5.0 g, 0.058 mol) was added to the dark red solution and the reaction was heated at reflux for 30 minutes. Cooled and concentrated under vacuum on a rotary evaporator, the crude product was purified by silica gel column chromatography with a gradient of 5% ethyl acetate, 95% hexane to 50% ethyl acetate 50% hexane over 20 minutes to afford 2.5 g of the title compound as a pale yellow oil. ¹H NMR (CDCl₃): δ 1.42 (t, 3H), 1.62 (d, 3H), 1.96 (s, 3H), 3.94 (q, 1H), 4.43 (q, 2H), 8.08 (s, 1H), and 8.71 (s, 1H).

### Step 3. Preparation of 5-(1-Methylthioethyl)-2-trifluoromethyl-nicotinic acid

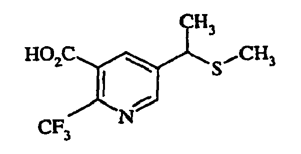

To a dry 50 mL glass vial equipped with magnetic stirrer, and nitrogen inlet, was charged 0.5 g (0.00170 moles) of 5-(1-methylthioethyl)-2-trifluoromethylnicotinic acid ethyl ester, and 10 mL of tetrahydrofuran (THF). The solution was cooled to 0°C and 5.1 mL of 1N aqueous lithium hydroxide solution (0.00511 moles) was added slowly via syringe. The reaction was stirred at 0°C for 1 hour, then overnight at ambient temperature. An aliquot was analyzed by thin layer chromatography (TLC) and high performance liquid chromatography (HPLC), which indicated that the reaction was essentially complete. The mixture was acidified to pH=2 with 1M aqueous hydrochloric acid, and extracted with 3 aliquots of 50 mL of ethyl acetate. The extract was dried over anhydrous magnesium sulfate (MgSO4), filtered, and concentrated under vacuum on a rotary evaporator to afford 0.410 g of the title compound as a tan solid. ¹H NMR (CDCl₃): δ 1.66 (d, 3H), 1.96 (s, 3H), 3.98 (q, 1H), 8.01 (bs, 1H), 8.30 (s, 1H), and 8.80 (s, 1H).

### Step 4. Preparation of 5-(1-Methylthioethyl)-2-trifluoromethylpyridine

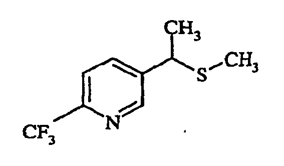

To a dry 50 mL round bottom flask equipped with magnetic stirrer, nitrogen inlet, thermometer, and reflux condenser was charged 0.35 g (0.00132 moles) of 5-(1-methylthioethyl)-2-trifluoromethylnicotinic acid, 0.17 g (0.00264 moles) of copper powder and 10 mL of DowTherm A. The reaction was heated at 240°C for 1 hour, then cooled to room temperature. The reaction mixture was extracted with 3 aliquots of 50 mL of ethyl acetate, and washed with 50 mL of water and 50 mL of saturated aqueous sodium chloride solution. The organic extract was dried over anhydrous magnesium sulfate, filtered, and concentrated under vacuum on a rotary evaporator. The crude product thus obtained was chromatographed on silica gel with a gradient of 100% hexane to 50% ethyl acetate, 50 % hexane. The pure fractions were combined, and concentrated under vacuum on a rotary evaporator to afford 0.13 g of the title compound as a pale yellow oil. ¹H NMR (CDCl₃): δ 1.62 (d, 3H), 1.94 (s, 3H), 3.93 (q, 1H), 7.68 (d, 1H), 7.90 (d, 1H), and 8.66 (s, 1H).

## Claims

1. A process for the preparation of 2-substituted-5-(1-alkylthio)-alkylpyridine (I), 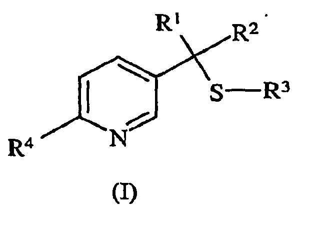 wherein
R¹ and R² independently represent H, C₁-C₄ alkyl, or either of R¹ or R² taken together with R³ represent a 4- to 6-membered saturated ring, or R¹ taken together with R² represent a 3- to 6-membered saturated rin optionally substituted with an O or a N atom g;
R³ represents C₁-C₄ alkyl or R³ taken together with either of R¹ or R² represent a 4- to 6-membered saturated ring; and
R⁴ represents C₁-C₄ alkyl or C₁-C₄ haloalkyl;
which comprises:
a) condensing a substituted enone (II), 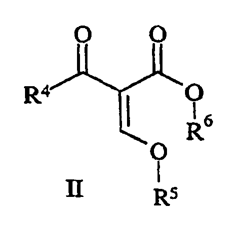 wherein
R⁴ is as previously defined; and
R⁵ and R⁶ independently represent C₁-C₄ alkyl;
with an enamine (III), 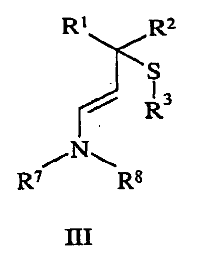 wherein
R¹, R² and R³ are as previously defined; and
R⁷ and R⁸ independently represent C₁-C₄ alkyl or R⁷ and R⁸ taken together with N represent a 5-membered saturated or unsaturated ring;
b) cyclizing the reaction mixture from step a) in the presence of ammonia or a reagent capable of generating ammonia to produce a 2,3,5-substituted pyridine (IV), 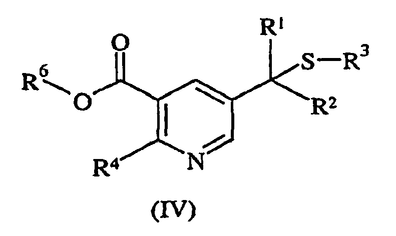 wherein
R¹, R², R³, R⁴ and R⁶ are as previously defined; and
c) saponifying and decarboxylating the 2,3,5-substituted pyridine (IV) to give 2-substituted-5-(1-alkylthio)alkylpyridine (I).

2. The process of Claim 1 in which R⁴ represents CF₃.

3. The process of Claim 2 in which R¹ represents H, R² represents CH₃, and R³ represents CH₃.

4. A compound of the Formula (IV) 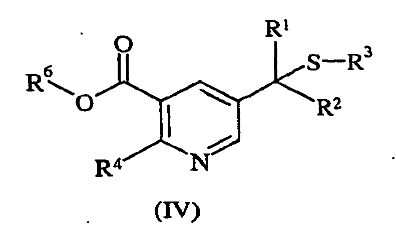 wherein
R¹ and R² independently represent H, C₁-C₄ alkyl, or either of R¹ or R² taken together with R³ represent a 4- to 6-membered saturated ring, or R¹ taken together with R² represent a 3- to 6-membered saturated ring optionally substituted with an O or a N atom;
R³ represents C₁-C₄ alkyl or R³ taken together with either of R¹ or R² represent a 4- to 6-membered saturated ring;
R⁴ represents C₁-C₄ alkyl or C₁-C₄ haloalkyl; and
R⁶ represents H or C₁-C₄ alkyl.

5. A compound of Claim 4 in which R⁴ represents CF₃.

## Patentansprüche

1. Verfahren zur Herstellung eines 2-substituierten 5-(1-Alkylthio)-alkylpyridins (I), 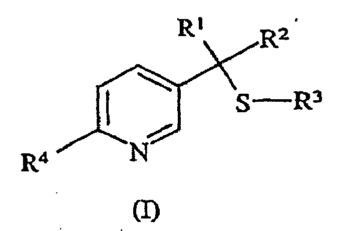 worin
R¹ und R² unabhängig H, C₁-C₄-Alkyl darstellen, oder eines von R¹ oder R² zusammen mit R³ einen 4- bis 6-gliedrigen gesättigten Ring darstellt, oder R¹ zusammen mit R² einen 3- bis 6-gliedrigen gesättigten Ring, optional substituiert mit einem O- oder einem N-Atom, darstellt;
R³ C₁-C₄-Alkyl darstellt, oder R₃ zusammen mit einem von R¹ oder R² einen 4- bis 6-gliedrigen gesättigten Ring darstellt; und
R⁴ C₁-C₄-Alkyl oder C₁-C₄-Haloalkyl darstellt;
umfassend:
a) Kondensieren eines substituierten Enons (II) 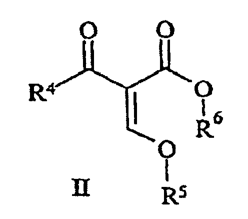 worin
R⁴ wie oben definiert ist; und
R⁵ und R⁶ unabhängig C₁-C₄-Alkyl darstellen;
mit einem Enamin (III), 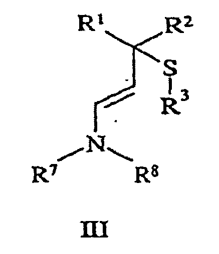 worin
R¹, R² und R³ wie oben definiert sind; und
R⁷ und R⁸ unabhängig C₁-C₄-Alkyl darstellen oder R⁷ und R⁸ zusammen mit N einen 5-gliedrigen gesättigten oder ungesättigten Ring darstellen;
b) Zyklisieren des Reaktionsgemischs von Schritt a) in der Gegenwart von Ammoniak oder eines Reagens, das Ammoniak erzeugen kann, um ein 2,3,5-substituiertes Pyridin (IV) herzustellen, 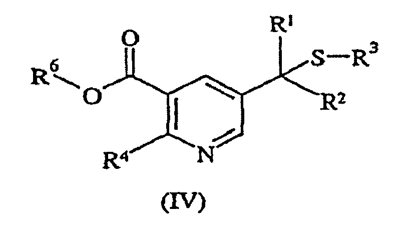 worin
R¹, R², R³, R⁴ und R⁶ wie oben definiert sind; und
c) Verseifen und Decarboxylieren des 2,3,5-substituierten Pyridins (IV), um 2-substituiertes 5-(1-Alkylthio)alkylpyridin (I) zu ergeben.

2. Verfahren nach Anspruch 1, worin R⁴ CF₃ darstellt.

3. Verfahren nach Anspruch 2, worin R¹ H darstellt, R² CH₃ darstellt und R³ CH₃ darstellt.

4. Verbindung der Formel (IV) 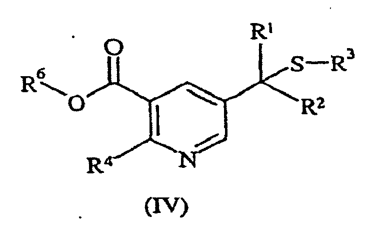 worin
R¹ und R² unabhängig H, C₁-C₄-Alkyl darstellen, oder eines von R¹ oder R² zusammen mit R³ einen 4- bis 6-gliedrigen gesättigten Ring darstellt, oder R¹ zusammen mit R² einen 3- bis 6-gliedrigen gesättigten Ring, optional substituiert mit einem O- oder einem N-Atom, darstellt;
R³ ein C₁-C₄-Alkyl darstellt, oder R₃ zusammen mit einem von R¹ oder R² einen 4- bis 6-gliedrigen gesättigten Ring darstellt;
R⁴ C₁-C₄-Alkyl oder C₁-C₄-Haloalkyl darstellt; und
R⁶ H oder C₁-C₄-Alkyl darstellt.

5. Verbindung nach Anspruch 4, worin R⁴ CF₃ darstellt.

## Revendications

1. Procédé de préparation d'une 5-(1-alkylthio-alkyl)-pyridine à substituant en position 2, de formule (I) : 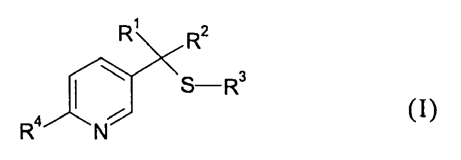 dans laquelle
- R¹ et R² représentent chacun, indépendamment, un atome d'hydrogène ou un groupe alkyle en C₁₋₄,
ou l'un ou l'autre de R¹ et R² représente, conjointement avec R³,
un cycle saturé de 4 à 6 chaînons,
ou R¹ et R² représentent conjointement un cycle saturé de 3 à 6 chaînons dont l'un peut, en option, être remplacé par un atome d'oxygène ou d'azote,
- R³ représente un groupe alkyle en C₁₋₄,
ou R³ représente, conjointement avec l'un ou l'autre de R¹ et R², un cycle saturé de 4 à 6 chaînons,
- et R⁴ représente un groupe alkyle en C₁₋₄ ou halogéno-alkyle en C₁₋₄,
lequel procédé comporte les étapes suivantes :
a) condenser une énone à substituants, de formule (II) : 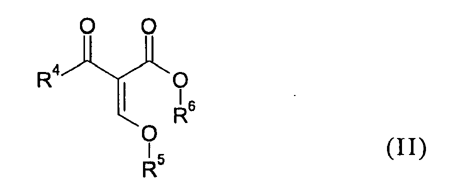 dans laquelle
- R⁴ a la signification indiquée ci-dessus,
- et R⁵ et R⁶ représentent chacun, indépendamment, un groupe alkyle en C₁₋₄,
avec une énamine de formule (III) : 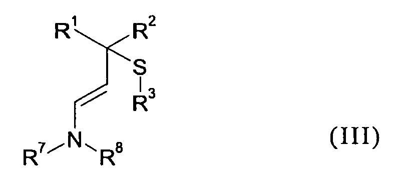 dans laquelle
- R¹, R² et R³ ont les significations indiquées ci-dessus,
- et R⁷ et R⁸ représentent chacun, indépendamment, un groupe alkyle en C₁₋₄,
ou R⁷ et R⁸ représentent, conjointement avec l'atome d'azote,
un cycle saturé ou insaturé de 5 chaînons ;
b) soumettre le mélange réactionnel issu de l'étape (a) à une cyclisation, en présence d'ammoniac ou d'un réactif capable de générer de l'ammoniac, afin d'obtenir une pyridine portant des substituants en positions 2, 3 et 5, de formule (IV) : 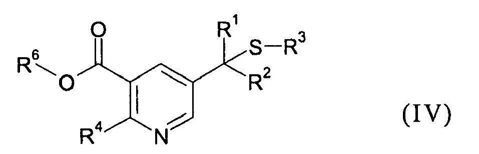 dans laquelle R¹, R², R³, R⁴ et R⁶ ont les significations indiquées ci-dessus ;
c) et soumettre cette pyridine portant des substituants en positions 2, 3 et 5, de formule (IV), à une saponification et à une décarboxylation, ce qui donne une 5-(1-alkylthio-alkyl)-pyridine à substituant en position 2, de formule (I).

2. Procédé conforme à la revendication 1, dans lequel R⁴ représente un groupe trifluorométhyle.

3. Procédé conforme à la revendication 2, dans lequel R¹ représente un atome d'hydrogène, R² représente un groupe méthyle, et R³ représente un groupe méthyle.

4. Composé de formule (IV) : 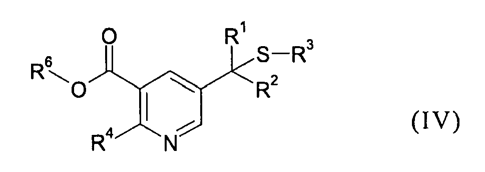 dans laquelle :
- R¹ et R² représentent chacun, indépendamment, un atome d'hydrogène ou un groupe alkyle en C₁₋₄,
ou l'un ou l'autre de R¹ et R² représente, conjointement avec R³,
un cycle saturé de 4 à 6 chaînons,
ou R¹ et R² représentent conjointement un cycle saturé de 3 à 6 chaînons dont l'un peut, en option, être remplacé par un atome d'oxygène ou d'azote,
- R³ représente un groupe alkyle en C₁₋₄,
ou R³ représente, conjointement avec l'un ou l'autre de R¹ et R²,
un cycle saturé de 4 à 6 chaînons,
- R⁴ représente un groupe alkyle en C₁₋₄ ou halogéno-alkyle en C₁₋₄,
- et R⁶ représente un atome d'hydrogène ou un groupe alkyle en C₁₋₄.

5. Composé conforme à la revendication 4, dans lequel R⁴ représente un groupe trifluorométhyle.
